# EUROPEAN PATENT SPECIFICATION

(11) **EP 3 973 027 B1**
(45) Date of publication and mention of the grant of the patent: **22.01.2025**
(21) Application number: 19929686.4
(22) Date of filing: 21.05.2019
(51) Int. Cl.: C09J 123/08, C09J 153/00, C08L 51/06, C08L 91/06, C08L 23/08, C08L 91/00

(54) **OLEFIN BLOCK COPOLYMER-BASED HOT MELT ADHESIVE COMPOSITION**
SCHMELZKLEBSTOFFZUSAMMENSETZUNG AUF OLEFINBLOCKCOPOLYMERBASIS
COMPOSITION ADHÉSIVE THERMOFUSIBLE À BASE DE COPOLYMÈRE SÉQUENCÉ D'OLÉFINE

(43) Date of publication of application: 30.03.2022
(73) Proprietor: Henkel AG & Co. KGaA, 40589 Düsseldorf (DE)
(72) Inventor: CHEN, MinRui, Shanghai 201615 (CN); LI, Weiyi, Foshan Guangdong 528216 (CN)
(86) International application number: PCT/CN2019/087757
(87) International publication number: WO 2020/232624

(56) References cited:
- WO-A1-2012/139120
- WO-A1-2015/102989
- WO-A1-2019/003964
- WO-A2-2011/014714
- WO-A2-2011/014714

## Description

### Technical field

The present invention relates to a hot melt adhesive composition, particularly, relates to an olefin block copolymer-based hot melt adhesive composition, especially used for disposable articles.

### Background of the invention

Hot melt adhesives have been widely used in disposable articles like baby diapers and feminine napkins, which comprise multiple layers such as a PE film, a hydrophobic non-woven layer, a hydrophilic non-woven layer, stretchable TPU film, tissue, cotton and the like. The layers are bonded together with the hot melt adhesive. Disposable articles usually comprise a core part for absorbing body fluids, and the core part often is composed of hydrophilic substrates as a wrapping material, and inside which is fluff and super absorbent polymer (SAP). The core part is usually soaked in body fluids and thus the adhesive used in the core part requires a high bonding strength under both dry and wet conditions. Otherwise, delamination of the layers will lead to leakage of body fluids, causing product quality issues. Therefore, a high wet peel strength for the hot melt adhesive used here is very important.

Typically, hot melt adhesives can be based on polymers such like functionalized or non-functionalized polyolefins, or styrenic block copolymers such as styrene-isoprene-styrene (SIS) or styrene-butadienestyrene (SBS) polymers.

In the past, many different olefinic polymers have been used in the hot melt adhesives for disposable articles. One important kind of the olefinic polymers is amorphous polyolefins (APO), such as amorphous polypropylene (APP). Later, polymers having much improved properties over the original APO polymers became available, such as amorphous poly alpha olefins (APAO).

WO2011/014714A2 (Henkel Corporation) discloses a low application temperature hot melt adhesive comprising a polyolefin copolymer component and a maleated polyethylene wax component. The polyolefin copolymer component comprises two different polyolefin copolymers wherein at least one polyolefin copolymer is a metallocene-catalyzed ethylene/α-olefin copolymer prepared from ethylene and 1-octene monomers, which copolymer that has a Melt Index of greater than about 400 g/10 min at 190°C. The adhesive has a viscosity at 120°C of below about 3000 centipoises.

WO2019/003964 A1 (Henkel AG & Co. KGaA) discloses a hot melt adhesive comprising: (A) a metallocene based propylene homopolymer; (B) a metallocene based ethylene/α-olefin copolymer; (C) a tackifier resin, and (D) a plasticizer, wherein the plasticizer (D) comprises (D1) an oil and (D2) at least one polymer selected from polybutene, polybutadiene, polyisobutylene, and polyisoprene.

Traditional APOs are homopolymers or copolymers of ethylene, propene, butene, monoene with longer aliphatic chains. In this case, physical performances like green strength, elasticity, resistance to elevated temperature of APO are not that good, because the molecular structure of APOs is quite random.

Spray uniformity is another concern for the hot melt adhesive used in the disposable articles. For application, the adhesive is firstly heated to become liquid, and then spray coated or slot coated onto substrates. The spray nozzle can be designed into specific shapes to create different spray patterns including summit, signature, spiral, curtain, omega etc. The spray process requires the hot melt adhesive to be featured with excellent spray uniformity. Traditional polyolefin has poor spray uniformity, which means the spray pattern is not even or regular as what it should be like. Uniform spray pattern is very important for creating suitable bonding effects, sometimes bad spray patterns will lead to bonding failure and cause serious issues.

The hot melt adhesives used in the disposable articles are desired to have a wide application window. The term 'application window' means various application conditions in which hot melt adhesives are used well in manufacturing lines. For disposable articles, the application window refers to the conditions including spray temperature, air pressure, spray pattern, open time, setting time, green strength etc. It is very important for hot melt adhesives to have a broad application window to meet all the above requirements, in order to reduce machine downtime and scrap as well as to make adhesive suitable for various working conditions. For example, on a manufacturing line of baby diapers, the hot melt adhesive is spray coated from the nozzle onto the first substrate, then the substrate moves forward for 10 meters on the conveyor, requiring 2 seconds as the open time for bonding of this position. The hot melt adhesive should remain tacky within the open time and therefore after 2 seconds it is still able to bond to the second substrate. Meanwhile, the hot melt adhesive should have excellent green strength so that in some working positions the adhesive can have instant bonding effect after lamination and therefore no misposition would take place. Modern high-speed manufacturing lines of disposable articles run at a speed up to 400-500 meters per minute, once the hot melt adhesive does not fall in the application window, it is very likely that the bonding performance of hot melt adhesive cannot meet the requirement of continuous production. For disposable hygiene applications, traditional APO-based hot melt adhesives cannot meet one or more of these requirements.

Therefore, it still needs to improve the application performance of the hot melt adhesives used for disposable articles.

### Summary of the invention

To solve the above-mention problems, an object of the present invention is to provide a hot melt adhesive composition having one or more properties of good green strength, good odor, good wet bonding strength, and good spray uniformity, preferably having all the above properties.

After intensive studies, the inventors have found that the above problems may be solved with a hot melt adhesive composition as defined in claim 1 appended hereto and which comprises: (A) an olefin block copolymer of ethylene and octene having a melt index of 10 to 35 g/10 min @ 190°C, 2.16 kg, preferably 15 to 30 g/10 min @ 190°C, 2.16 kg; and, (B) a wax modified with carboxylic acid and/or carboxylic acid anhydride.

In the present invention, the wax (B) preferably is a polyolefin wax modified with maleic acid and/or maleic acid anhydride.

In the present invention, the wax (B) preferably is a polypropylene wax modified with maleic acid and/or maleic acid anhydride, or a polyethylene wax modified with maleic acid and/or maleic acid anhydride.

In the present invention, the wax (B) preferably has an acid number of no less than 10 mg KOH/g, preferably no less than 15 mg KOH/g.

The advantages of the hot melt adhesive composition include that:
- It is featured with excellent bonding strength under both dry and wet conditions, ensuring no leakage and no detachment in wet condition.
- It is featured with lower odor by selecting the components described herein, compared to SBS / SIS / EVA based hot melt adhesives.
- It is featured with a wide application window, including good elasticity, long open time, excellent green strength etc, which are very important for hot melt adhesives applied on disposable hygiene articles.
- It has good spray uniformity.
- Compared to traditional SIS / SBS based hot melt adhesives, the bonding strength of the adhesive of the present invention is even higher, which means the coating weight can be potentially lowered so as to reduce the consumption of hot melt adhesive.

### Detailed description of the invention

It is to be understood by one of ordinary skill in the art that the present invention is a description of exemplary embodiments only, and is not intended as limiting the broader aspects of the present invention. Each aspect so described may be combined with any other aspect or aspects unless clearly indicated to the contrary. In particular, any feature indicated as being preferred or advantageous may be combined with any other feature or features indicated as being preferred or advantageous.

Unless specified otherwise, in the context of the present invention, the terms used are to be construed in accordance with the following definitions.

Unless specified otherwise, all wt% or % by weight values quoted herein are percentages by weight based on total weight of the cyanoacrylate adhesive composition.

Unless specified otherwise, as used herein, the terms "a", "an" and "the" include both singular and plural referents.

The terms "comprising" and "comprises" as used herein are synonymous with "including", "includes" or "containing", "contains", and are inclusive or open-ended and do not exclude additional, non-recited members, elements or process steps.

Unless specified otherwise, the recitation of numerical end points includes all numbers and fractions subsumed within the respective ranges, as well as the recited end points.

Unless otherwise defined, all terms used in the present invention, including technical and scientific terms, have the meaning as commonly understood by one of the ordinary skilled in the art to which this invention belongs.

According to the present invention, the hot melt adhesive composition comprises: (A) an olefin block copolymer of ethylene and octene, having a melt index of 10 to 35 g/10 min @ 190°C, 2.16 kg, preferably 15 to 30 g/10 min @ 190°C, 2.16 kg, and (B) a wax modified with carboxylic acid and/or carboxylic acid anhydride.

### (A) Olefin block copolymer of ethylene and octene

An important component used in a hot melt adhesive formula according to the present invention is an olefin block copolymer (OBC).

OBCs are relatively new but known in the art. OBCs belong to a new class of polyolefin polymers produced using a chain shuttling catalysis technology that produces a linear block structure of the monomers rather than a random polymer produced by Ziegler-Natta or traditional metallocene technology. At present, they are mainly manufactured by Dow Chemical under the trade name of Infuse^{®}. The OBCs consist of crystallizable ethylene-octene blocks (hard) with very low comonomer content and high melting point alternating with amorphous ethylene-octene blocks (soft) with high comonomer content and low glass transition temperature. This gives the polymer much better elevated temperature resistance and elasticity compared to a typical metallocene random polymer of similar density. Details of these polymers and their synthesis and physical properties can be found in, for example, WO 2006/101966, WO 2006/102016, WO 2006/102150, WO 2009/029476 and U.S. Pat. No. 7,524,911, the disclosures of which are specifically incorporated herein by reference.

Specifically, the OBCs useful in the present invention comprise polymerized units of ethylene and octene, wherein the OBCs are characterized by an average block index greater than zero and up to about 1.0 and a molecular weight distribution, Mw/Mn, greater than about 1.3. Preferably, the OBCs useful in the present invention comprise polymerized units of ethylene and octene, wherein the average block index is greater than 0 but less than about 0.4 and a molecular weight distribution, Mw/Mn, greater than about 1.3. More preferably, the OBCs useful in the present invention are linear, multi-block copolymers with at least three blocks. More preferably, the ethylene content in the OBCs useful in the present invention is at least 50 mole percent.

In some embodiments, the average block index of the OBCs useful in the present invention is in the range from about 0.1 to about 0.3, from about 0.4 to about 1.0, from about 0.3 to about 0.7, from about 0.6 to about 0.9, or from about 0.5 to about 0.7. In other embodiments, the OBCs have a density of less than about 0.91 g/cc, such as from about 0.86 g/cc to about 0.91 g/cc. In other embodiments, the molecular weight distribution, Mw/Mn, is greater than about 1.5 or greater than about 2.0. It can also range from about 2.0 to about 8 or from about 1.7 to about 3.5.

Preferably, the OBCs useful in the present invention comprise at least one fraction obtained by Temperature Rising Elution Fractionation ("TREF"), wherein the fraction has a block index greater than about 0.3 and up to about 1.0, and the OBCs useful in the present invention have a molecular weight distribution, Mw/Mn, greater than about 1.3. Preferably, the OBCs useful in the present invention comprise polymerized units of ethylene and octene, the OBCs are characterized by having at least one fraction obtained by TREF, wherein the fraction has a block index greater than about 0 and up to about 0.4 and the OBCs have a molecular weight distribution, Mw/Mn, greater than about 1.3. In some embodiments, the block index of the fraction is greater than about 0.4, greater than about 0.5, greater than about 0.6, greater than about 0.7, greater than about 0.8, or greater than about 0.9.

The term "block index" used herein has the same meaning as defined in WO2006/101966.

Preferably, the OBCs useful in the present invention comprise one or more hard segments and one or more soft segments. Preferably, the hard segments comprise at least 98% of ethylene by weight., and the soft segments comprise less than 95%, preferably less than 50%, of ethylene by weight. In some embodiments, the hard segments are present in an amount from about 5% to about 85% by weight of the OBC. In other embodiments, the OBC comprises at least 5 or at least 10 hard and soft segments connected in a linear fashion to form a linear chain. Preferably, the hard segments and soft segments are randomly distributed along the chain. In some embodiments, neither the soft segments nor the hard segments include a tip segment (which is different by chemical composition than the rest of the segments).

Olefin block copolymers should not be considered as amorphous poly-alpha-olefins because the polymer architecture is completely different (i.e. block vs. random) and is specifically produced to have crystalline regions. In addition, OBCs are significantly narrower in polydispersity than other traditionally used olefins, for example APAOs, which impacts their melt profiles as measured by DSC (Differential Scanning Calorimetry).

Olefin block copolymers (OBCs) are polyolefins with alternating blocks of hard (highly rigid) and soft (highly elastomeric) segments. The block structure of OBCs offers an advantaged performance balance of flexibility and sprayability compared to random polyolefin copolymers. OBCs useful in the present invention can be commercially available from Dow Chemical Company under the tradenames Infuse^{®} 9807, Infuse^{®} 9817 and Infuse^{®} 9900.

Olefin block copolymers of ethylene and octene useful in the present hot melt adhesive composition preferably are olefin block copolymers of ethylene and 1-octene, more preferably have a melt index in the range of 10 to 35 g/10 min @ 190 °C, 2.16 kg, preferably 15 to 30 g/10 min @ 190 °C, 2.16 kg, as measured according to ASTM D1238, such as 12, 14, 16, 18, 20, 22, 24, 26, 28, 32, 34 g/10 min @ 190 °C, 2.16 kg, as determined according to ASTM D1238. A melt index outside the above range may lead to unsatisfactory effect.

Preferably, olefin block copolymers of ethylene and octene useful in the present hot melt adhesive composition have a melting point in the range of 110 °C to 130 °C, preferably, of 115 °C to 125 °C, such as 118 °C, 120 °C and 122 °C, as determined by differential scanning calorimetry (DSC).

One or more olefin block copolymers of ethylene and octene are incorporated into the adhesive composition in an amount of from 10% to 30% by weight, preferably from 10% to about 25% by weight, such as 12%, 15%, 18%, 20%, 22%, 27%, 29% by weight, each based on the total weight of the adhesive composition.

### (B) Wax modified with carboxylic acid and/or carboxylic acid anhydride

The wax (B) used in the present invention is not particularly limited, as long as it is modified with carboxylic acid and/or carboxylic acid anhydride and the objective hot melt adhesive of the present invention can be obtained.

Examples of the wax (B) include:
- a wax obtained by graft polymerization of carboxylic acid and/or carboxylic acid anhydride with a base wax, and
- a wax obtained by copolymerization of carboxylic acid and/or carboxylic acid anhydride in case of synthesizing the wax by polymerization.

The carboxylic acid and/or carboxylic acid anhydride functional groups can be introduced in the wax (B) via various reactions.

The "base wax" useful in the present invention is not particularly limited as long as it is a wax to be commonly used in a hot melt adhesive and the objective hot melt adhesive of the present invention can be obtained. Specific examples thereof include synthetic waxes such as a polyolefin wax (polyethylene wax, polypropylene wax); petroleum waxes such as a paraffin wax and a microcrystalline wax; and natural waxes such as a castor wax.

The carboxylic acid and/or carboxylic acid anhydride to be used to modify the base wax is/are not particularly limited as long as the objective hot melt adhesive of the present invention can be obtained. Specific examples thereof include maleic acid, maleic acid anhydride, fumaric acid, succinic acid, succinic acid anhydride, phthalic acid, phthalic acid anhydride, glutaric acid, glutaric acid anhydride, itaconic acid, acrylic acid, methacrylic acid and the like. These carboxylic acids and/or carboxylic acid anhydrides may be used alone, or in combination. In the present invention, maleic acid and/or maleic acid anhydride is/are particularly preferable.

In order to modify the base wax, it is possible to use various carboxylic acid derivatives which can introduce polar groups (i.e., carboxylic acid group and/or carboxylic acid anhydride group). Herein, "carboxylic acid derivatives" include:
- carboxylic acid esters such as ethyl acetate and vinyl acetate;
- acid halides such as benzoyl bromide;
- amides such as benzamide, N-methylacetamide and N,N- dimethylformamide ;
- imides such as succinimide;
- acyl azides such as acetyl azide;
- hydrazides such as propanoyl hydrazide;
- hydroxamic acids such as chloroacetylhydroxamic acid; lactones such as γ-butyrolactone ; and
- lactams such as δ-caprolactam.

The wax modified with carboxylic acid and/or carboxylic acid anhydride of the present invention is preferably a polyolefin wax modified with maleic acid and/or maleic acid anhydride, and particularly preferably a polypropylene wax modified with maleic acid anhydride or a polyethylene wax modified with maleic acid anhydride.

As (B) the wax modified with carboxylic acid and/or carboxylic acid anhydride, it is possible to use commercially available products in the present invention. Examples thereof include Licocene^{®} PPMA 6252 and PPMA 1332 available from Clariant; A-C 575P and A-C 597P available from Honeywell.

Preferably, the wax modified with carboxylic acid and/or carboxylic acid anhydride of the present invention (B) useful in the present invention has an acid number of no less than 10 mg KOH/g, preferably no less than 15 mg KOH/g, such as 15, 18, 20, 30, 50, 60, 70, 85, 88, 100 mg KOH/g, as determined according to DIN EN ISO 2114.

Preferably, the wax modified with carboxylic acid and/or carboxylic acid anhydride of the present invention (B) useful in the present invention has a softening point in the range of 70 °C to 150 °C, preferably in the range of 80 °C to 140 °C, such as 90 °C, 100 °C, 105 °C, 110 °C, 120 °C, 130 °C, as determined according to ASTM D 3954.

The hot melt adhesives of the present invention may comprise one or more waxes modified with carboxylic acid and/or carboxylic acid anhydride.

In the present invention, the blending amount of the wax (B) is preferably from 0.1 to 10 % by weight, preferably 0.2 to 8 % by weight, such as 0.5, 1, 1.5, 2, 2.5, 3, 3.5, 4, 4.5, 5, 5.5, 6, 6.5, 7, 7.5 % by weight, each based on the total weight of the adhesive. By blending the wax (B) in the above amount, the obtained hot melt adhesive has little odor while providing wet adhesion.

### Other components

In addition to the above component (A) and component (B), the adhesive composition of the present invention may further comprise (C) a tackifier (also called as tackifying resin).

The tackifier (C) is preferably blended in the amount within a range from 30 to 80 % by weight, more preferably from 40 to 70 % by weight, such as 50% by weight, 60% by weight, 65% by weight, each based on the total weight of the adhesive composition.

Any tackifying resins that are compatible in the formulation may be blended. Examples of the tackifier (C) include a natural rosin, a modified rosin, a hydrogenated rosin, a glycerol ester of a natural rosin, a glycerol ester of a modified rosin, a pentaerythritol ester of a natural rosin, a pentaerythritol ester of a modified rosin, a pentaerythritol ester of a hydrogenated rosin, a copolymer of a natural terpene, a terpolymer of a natural terpene, hydrogenated derivatives of a copolymer of a hydrogenated terpene, a polyterpene resin, hydrogenated derivatives of a phenol-based modified terpene resin, an aliphatic petroleum hydrocarbon resin, hydrogenated derivatives of an aliphatic petroleum hydrocarbon resin, an aromatic petroleum hydrocarbon resin, hydrogenated derivatives of an aromatic petroleum hydrocarbon resin, a cyclic aliphatic petroleum hydrocarbon resin, and hydrogenated derivatives of a cyclic aliphatic petroleum hydrocarbon resin. Synthetic aliphatic tackifiers are more preferred. More particularly, fully hydrogenated aliphatic tackifiers are even more preferred. Hydrogenated aliphatic C5 or dicyclopentadiene (DCPD) resins are fully compatible with SEBS and Polyolefin. These tackifiers can be used alone, or in combination. It is also possible to use a liquid type tackifier as long as it has a colorless to pale yellow color tone and substantially no odor, and also has satisfactory thermal stability. Taking these properties into consideration comprehensively, the tackifier is preferably hydrogenated derivatives of resins, and particularly preferably a hydrogenated dicyclopentadiene-based resin. Preferably, the tackifiers possess a Ring & Ball softening point of from 70 °C to 150 °C.

It is possible to use commercially available products as the tackifier (C). Examples of these commercially available products include C100R / C100W / H130R / H130W from Eastman, H5-1000 / 1001 from Henghe China, SU 90 / 100 / 120 /130 from Kolon, I-Marv P90 / P100 / P120 from Arakawa, Regalite R1100 / R1120 / S1100 from Eastman, Escorez 5300 / 5400 from Exxon, HA 103 from Tonen, HD 1100 / 1120 from Luhua, PRS-5100 / 5120 from Ruisen, JH-6100 / 6125 from Jin-hai. These commercially available tackifier resins can be used alone, or in combination.

The hot melt adhesive of the present invention can further include oil (D). The oil (D) is blended as a plasticizer for the purpose of decrease in melt viscosity and Tg of the hot melt adhesive, imparting of flexibility and improvement in wettability to an adherend. Examples of the oil (D) include mineral oils like paraffin oil, naphthene oil and aromatic oil, and colorless and odorless oils such as naphthene oil and paraffin oil are particularly preferable.

It is possible to use commercially available products as the oil (D). Examples thereof include KN 4006, KN 4008, KN 4010 from Lubricant Company of PetroChina; white mineral oil LP 150, LP 350 from Kukdong Korea; Nyflex 222B from Nynas; PS white mineral oil from PetroChina. These oils (D) can be used alone, or in combination.

If present, the oil (D) is preferably blended in the amount within a range from 5 to 35 % by weight, more preferably from 8 to 30 % by weight, such as 10% by weight, 15% by weight, 20% by weight, 25% by weight, each based on the total weight of the adhesive composition.

The hot melt adhesive of the present invention can further include stabilizer (E). The "stabilizer" is blended so as to improve stability of the hot melt adhesive by preventing decrease in molecular weight, gelation, coloration, and generation of odor of the hot melt adhesive due to heat, and there is no particular limitation on the stabilizer as long as the objective hot melt adhesive of the present invention can be obtained. In the present invention, the term "stabilizer" cover antioxidants and ultraviolet absorbers. The "ultraviolet absorber" is used so as to improve light resistance of the hot melt adhesive. The "antioxidant" is used so as to prevent oxidation degradation of the hot melt adhesive. The antioxidant and ultraviolet absorber are commonly used in the hot melt adhesive for disposable products and can be used without particular limitation as long as the objective disposable products can be obtained.

Examples of the antioxidant include a phenol-based antioxidant, a sulfur-based antioxidant and a phosphorus-based antioxidant. Examples of the ultraviolet absorber include a benzotriazole-based ultraviolet absorber and a benzophenone-based ultraviolet absorber. It is also possible to add a lactone-based stabilizer. These stabilizers can be used alone, or in combination.

It is possible to use commercially available products as the stabilizer. Examples thereof include Evernox 10GF / 1726 from Everspring, Irganox 1010/ 1726 from BASF, Thanox 1010G / 1726 from Rianlon; Commercial grades of phosphite include Everfos 168 from Everspring, Irgafos 168 from BASF, Thanox 168 from Rianlon; Commercial grades of thiodipropionate include Thanox 412S / DSTP from Rianlon, ADK AO 412S from Adeka, Sumilizer TP-D from Sumimoto. These stabilizers can be used alone, or in combination.

The stabilizer (E) can be contained in the adhesive composition of the present invention in an amount of less than 5 wt%, preferably less than 3 wt%, more preferably less than 2 wt%, such as about 1 wt%.

The hot melt adhesive composition of the present invention can further include a filler, especially a fine particle filler. Commonly used fine particle filler may be used, and there is no particular limitation as long as the objective hot melt adhesive of the present invention can be obtained. Examples of the "fine particle filler" include mica, calcium carbonate, kaolin, talc, titanium oxide, diatomaceous earth, urea-based resin, styrene beads, calcined clay, starch and the like. These particles preferably have a spherical shape, and there is no particular limitation on the size (diameter in case of a spherical shape) .

The hot melt adhesive composition of the present invention can further include other additives commonly used in the art, such as pigments, dyes, rheology modifiers, and so on.

The hot melt adhesive of the present invention can be produced by blending the components (A) and (B) and other optional components, melting the mixture under high temperature e.g. above 140 °C, followed by mixing or homogenizing to form an uniform mixture. Any feasible blending methods or equipments that are known in the art can be adopted to prepare a homogeneous adhesive mixture.

The hot melt adhesive according to the present invention preferably has a melt viscosity at 160°C from 2,000 to 3,500 mPa.s , and particularly preferably from 2,500 to 3,200 mPa.s. The "melt viscosity" is viscosity of the hot melt adhesive which is molten, and is measured by a Brookfield RVT-type viscometer (spindle No. 27).

As mentioned above, the hot melt adhesive according to the present invention can be employed in disposable products since it is excellent in adhesion in a wet state. The disposable products can be constituted by coating at least one kind of member selected from a group consisting of a woven fabric, a nonwoven fabric, a rubber, a resin, papers and a polyolefin film with the hot melt adhesive according to the present invention. The polyolefin film is preferably a polyethylene film for the reason of durability, costs and the like.

There is no particular limitation on the disposable products as long as they are so-called sanitary materials. Specific examples thereof include a disposable diaper, a sanitary napkin, a bed pad, a bandage, a surgical drape, a pet sheet, a hospital gown, a surgical white garment and the like.

In the production line of the disposable products, various members (for example, tissue, cotton, nonwoven fabric, polyolefin film, etc.) of the disposable products are commonly coated with the hot melt adhesive. In case of coating, the hot melt adhesive may be discharged (or ejected) from various dischargers (or ejectors) .

There is no particular limitation on the method of coating with the hot melt adhesive as long as the objective disposable products can be obtained. Such a coating method is roughly classified into a contact coating method and a non-contact coating method. The "contact coating" method refers to a coating method in which a discharger is brought into contact with a member or a film in case of coating with the hot melt adhesive, while the "non-contact coating" method refers to a coating method in which a discharger is not brought into contact with a member or a film in case of coating with the hot melt adhesive. Examples of the contact coating method include a slot coater coating method, a roll coater coating method and the like, and examples of the non-contact coating method include a spiral coating capable of coating in a spiral form, an omega coating or control seam coating method capable of coating in a wavy form, a slot spray coating or curtain spray coating method capable of coating in a plane form, and dot coating capable of coating in a dot form.

In another aspect of the present invention, provided is a laminate, comprising a first substrate, a second substrate, and an adhesive layer sandwiched therebetween, wherein the first and second substrates are independently of each other selected from a woven fabric, a nonwoven fabric, a rubber, a resin, papers and a polyolefin film, and the adhesive layer is formed by the hot melt adhesive composition according to the present invention.

In yet another aspect of the present invention, provided is a disposable article comprising the laminate of the present invention or produced using the hot melt adhesive of the present invention.

### Examples

The following examples are intended to assist one skilled in the art to better understand and practice the present invention. The scope of the invention is not limited by the examples but is defined in the appended claims. All parts and percentages are based on weight unless otherwise stated.

### Raw materials:

A1: Infuse 9807 is an OBC of ethylene/octene, with a melting point of 118°C and a melt index of 15 g/10min @ 190°C, 2.16kg. It is available from Dow.
A2: Infuse 9817 is an OBC of ethylene/octene, with a melting point of 120°C and a melt index of 15 g/10min @ 190°C, 2.16kg. It is available from Dow.
A3: Infuse 9900 is an OBC of ethylene/octene, with a melting point of 122°C and a melt index of 30 g/10min @ 190°C, 2.16kg. It is available from Dow.
A4': Infuse 9507 is an OBC of ethylene/octene, with a melting point of 119°C and a melt index of 5 g/10min @ 190°C, 2.16kg. It is available from Dow.
A5': Affinity 1950 is a metallocene crystalline ethylene/octene copolymer, with a melting point of 100°C and a melt index of 500 g/10min @ 190°C, 2.16kg. It is available from Dow.
A6': Vestoplast 704 is an amorphous propylene/butene-based polyolefin copolymer, with a melting point of 105 °C, available from Evonik.
A7': L Modu S400 is metallocene propylene-based polyolefin, with a softening point of 93°C and a melt viscosity of 8000cps @ 190°C. It is available from Idemitsu.
A8': Aerafin 17 is a propylene-based olefin polymer, , with a softening point of 130°C and a melt viscosity of 1700cps @ 190°C. It is available from Eastman.
A9': Vector 4113N is a synthetic SIS block copolymer with a styrene content of 15% and deblock content of 18%. It is available from TSRC.
B1: PPMA 1332 is a synthetic polypropylene wax grafted with maleic acid anhydride. It has a softening point of 80 °C and acid number of 17mgKOH/g. It is available from Clariant
B2: PPMA 6252 is a synthetic polypropylene wax grafted with maleic acid anhydride. It has a softening point of 140 °C and acid number of 40mgKOH/g. It is available from Clariant
B3: A-C 575P is a synthetic polyethylene wax grafted with maleic acid anhydride. It has a softening point of 106 °C and acid number of 35mgKOH/g. It is available from Honeywell.
B4': Sasolwax H1 is a Fischer Tropsch wax, with a melting point of 100°C, available from Sasol Wax.
C1: SU 100 is a hydrogenated DCPD aliphatic petroleum tackifier, with a softening point of 100°C. It is available from Kolon.
D1: LP 350 is a colourless, viscous mineral oil available from Kukdong Korea.
E1: Evernox 10GF is an antioxidant from Everspring.
E2: Everfos 168GF is an antioxidant from Everspring.

### Test Methods:

### Softening Point:

The soften point of the hot melt adhesive composition was determined according to ASTM D 3954.

### Odor:

The odor score of the hot melt adhesive composition was determined by the steps of:
a) placing the hot melt adhesive composition in a closed container;
b) heating the container to 160 °C for 2 hours;
c) cooling the container to 25 °C;
d) reheating the container to 70 °C for 1 hour;
e) cooling the container to 25 °C;
f) opening the container and organizing a group of test panel members to smell the odor from the container at a distance of 5 cm from the opening of the container;
g) assigning a score of the hot melt adhesive by each of the test panel member independently according to the rules below:
   - a hot melt adhesive with no odor was assigned with a score of 1;
   - a hot melt adhesive with slight but detectable odor was assigned with a score of 2;
   - a hot melt adhesive with some odor but not strong was assigned with a score of 3;
   - a hot melt adhesive with strong odor was assigned with a score of 4;
   - a hot melt adhesive with pungent odor was assigned with a score of 5; and
h) obtaining an average score of the hot melt adhesive from all the test panel members as the odor score for the hot melt adhesive.

An odor score of greater than 2.5 is considered as unacceptable during use.

### Green strength:

30-micron thickness adhesive were coated on a 50micron PET film with a film coater (laminated with a release film). Let it dwell at room temperature for 24h, then cut it into a 1 inch width and 5 inch length, hot pressed the adhesive film onto another 50micron PET film at 160 °C, pressure was 0.5MPa, pressing time was 5s. Then took the sample to the tensile tester to test the peel strength immediately. The test was started within 15s after hot pressing. The tensile test was done in the environment of 50 °C with an environmental box.
- Peel strength higher than 3N was judged as good;
- Peel strength between 1.5N and 3N was judged as medium;
- Peel strength lower than 1.5N was judged as bad.

### Spray uniformity:

The hot melt adhesive was molten at 160°C, and summit sprayed onto a substrate, the spray pattern was observed visually, and an uniform summit pattern was assigned as good, a nonuniform summit pattern was assigned as bad.

### Peel strength by summit spray:

A PE sheet (Air permeable cast film with a thickness of 18 gsm from Foshan Landi) and a tissue (cellulose tissue with a thickness of 18 gsm from Zhongzhi) were adhered together to form a laminate sample by applying the hot melt adhesive between the two sheets via Summit nozzle head. The PE sheet and the tissue were peeled from one another at an angle of 180° and with a rate of 300 mm/min using Instron 3365 tensile strength tester (for wet peel, additionally soak the sample in water for 3mins and then take it out to test). The T peel test was carried out at 25°C and 50% relative humidity. The T peel test was made to the laminate sample at least 72 hours after the hot melt adhesive was applied. The PE sheet and the tissue were pulled 10 cm apart and the force was recorded as the T peel strength of the hot melt adhesive for PE and tissue.

As for the Cotton NW, the same procedure was conducted to determine the dry and wet peel strengths.

### Examples 1 to 7 and Comparative Examples 1 to 7

Adhesives were prepared with conventional method in the art using components in amounts (parts by weight) listed in the Table 1, and the properties were tested using the methods stated above, and the results of evaluations are shown in Table 1.

**Table 1:**

| Ingredients | CE.1 | CE.2 | CE.3 | CE.4 | CE.5 | CE. 6 | CE.7 |
|---|---|---|---|---|---|---|---|
| (A1): Infuse 9807 | 18 | 18.5 | | | | | |
| (A2): Infuse 9817 | | | | | | | |
| (A3): Infuse 9900 | | | | | | | |
| (A4'): Infuse 9507 | | | | | | 18 | |
| (A5'): Affinity 1950 | | | | | | | 18 |
| (A6'): Vestoplast 704 | | | | 30 | | | |
| (A7'): L Modu 5400 | | | 30 | | | | |
| (A8'): Aerafin 17 | | | 20 | 20 | | | |
| (A9'): Vector 4113N | | | | | 19 | | |
| (B1): PPMA 1332 | | | | 2 | 2 | 2 | 2 |
| (B2): PPMA 6252 | | | 2 | | | | |
| (B3): A-C 575P | | | | | | | |
| (B4'): Sasolwax H1 | 2 | | | | | | |
| (C1): SU 100 | 60 | 60 | 37 | 37 | 60 | 60 | 60 |
| (D1): LP 350 | 19 | 20.5 | 10 | 10 | 18 | 19 | 19 |
| (E1): Evemox 10GF | 0.5 | 0.5 | 0.5 | 0.5 | 0.5 | 0.5 | 0.5 |
| (E2): Everfos 168GF | 0.5 | 0.5 | 0.5 | 0.5 | 0.5 | 0.5 | 0.5 |
| | | | | | | | |
| Viscositv(cps) at 160°C | 2950 | 3040 | 3200 | 2200 | 2600 | 7800 | 200 |
| Softening Point (°C) | 109 | 103 | 88 | 101 | 90 | 106 | 65 |
| Odor | 2 | 2 | 2.2 | 2.1 | 2.6 | 2.1 | 2.1 |
| | | | | | | | |
| Green strength | good | bad | bad | bad | good | good | bad |
| Spray uniformity | good | good | good | bad | good | bad | bad |

| Peel strength by summit spray | | | | | | | |
|---|---|---|---|---|---|---|---|
| PE / Cotton NW | 37.40 (0.95) | 35.04 (0.89) | 29.53 (0.75) | 27.95 (0.71) | -- | 35.83 (0.91) | <7.9 (<0.2) |
| N/m, 2 gsm, dry (N/inch, 2gsm, dry) | | | | | | | |
| PE / Cotton NW | 4.72 (0.12) | 4.33 (0.11) | 11.42 (0.29) | 11.02 (0.28) | -- | 29.92 (0.76) | <3.9 (<0.1) |
| N/m, 2 gsm, wet (N/inch, 2gsm, wet) | | | | | | | |
| PE / tissue | 24.80 (0.63) | 25.23 (0.59) | 18.90 (0.48) | 17.32 (0.44) | -- | 2402 (0.61) | <7.9 (<0.2) |
| N/m, 2 gsm, dry (N/inch, 2gsm, dry) | | | | | | | |
| PE / tissue | 1.97 (0.05) | 2.76 (0.07) | 8.27 (0.21) | 6.69 (0.17) | -- | 14.96 (0.38) | <3.9 (<0.1) |
| N/m, 2 gsm, wet (N/inch, 1.2gsm, wet) | | | | | | | |

**Table 1 (continued):**

| Ingredients | Ex.1 | Ex.2 | Ex. 3 | Ex.4 | Ex. 5 | Ex.6 | EX.7 |
|---|---|---|---|---|---|---|---|
| (A1): Infuse 9807 | 18 | 18 | 18 | 9 | | 18 | |
| (A2): Infuse 9817 | | | | 9 | 18 | | |
| (A3): Infuse 9900 | | | | | | | 18 |
| (A4'): Infuse 9507 | | | | | | | |
| (A5'): Affinity 1950 | | | | | | | |
| (A6'): Vestoplast 704 | | | | | | | |
| (A7'): L Modu 5400 | | | | | | | |
| (A8'): Aerafin 17 | | | | | | | |
| (A9'): Vector 4113N | | | | | | | |
| (B1): PPMA 1332 | 2 | | | 2 | 2 | 4 | 2 |
| (B2): PPMA 6252 | | 2 | | | | | |
| (B3): A-C 575P | | | 2 | | | | |
| (B4'): Sasolwax H1 | | | | | | | |
| (C1): SU 100 | 60 | 60 | 60 | 60 | 60 | 60 | 60 |
| (D1): LP 350 | 19 | 19 | 19 | 19 | 19 | 17 | 19 |
| (E1): Evemox 10GF | 0.5 | 0.5 | 0.5 | 0.5 | 0.5 | 0.5 | 0.5 |
| (E2): Everfos 168GF | 0.5 | 0.5 | 0.5 | 0.5 | 0.5 | 0.5 | 0.5 |
| | | | | | | | |
| Viscosity(cps) at 160°C | 3040 | 3000 | 3120 | 3010 | 2970 | 3120 | 2610 |
| Softening Point(°C) | 103 | 112 | 110 | 104 | 105 | 104 | 105 |
| Odor | 2.1 | 2.2 | 2.2 | 2.1 | 2.1 | 2.2 | 2.1 |
| | | | | | | | |
| Green strength | good | good | good | good | good | good | good |
| Spray uniformity | good | good | good | good | good | good | good |

| Peel strength by summit spray | | | | | | | |
|---|---|---|---|---|---|---|---|
| PE / Cotton NW | 38.19 (0.97) | 36.61 (0.93) | 37.80 (0.96) | 35.4 (0.9) | 33.86 (0.86) | 38.98 (0.99) | 31.89 (0.81) |
| N/m, 2 gsm, dry (N/inch, 2gsm, dry) | | | | | | | |
| PE / Cotton NW | 32.28 (0.82) | 31.10 (0.79) | 31.5 (0.8) | 30.31 (0.77) | 29.13 (0.74) | 34.65 (0.88) | 27.05 (0.71) |
| N/m, 2 gsm, wet (N/inch, 2gsm, wet) | | | | | | | |
| PE / tissue | 25.59 (0.65) | 2402 (0.61) | 27.6 (0.7) | 22.44 (0.57) | 21.26 (0.54) | 26.38 (0.67) | 26.38 (0.67) |
| N/m, 2 gsm, dry (N/inch, 2gsm, dry) | | | | | | | |
| PE / tissue | 16.93 (0.43) | 15.8 (0.4) | 16.14 (0.41) | 11.8 (0.3) | 11.42 (0.29) | 21.65 (0.55) | 19.7 (0.5) |
| N/m, 2 gsm, wet (N/inch, 1.2gsm, wet) | | | | | | | |

As can be seen from Table 1, the adhesives of the present invention (Ex. 1 to 7) showed good green strength, good odor, good wet bonding strength and good spray uniformity.

Comparative examples 1 to 2 (CE. 1 and CE. 2) did not use MA grafted wax, Comparative examples 3 to 5 and 7 (CE. 3 to CE. 5 and CE. 7) did not use OBC, and Comparative example 6 (CE. 6) used MA grafted wax and OBC with MI outside of the range of 10 to 35 g/10 min @ 190 °C, 2.16 kg, they all show one or more unsatisfied properties compared with the adhesives of the present invention.

## Claims

1. A hot melt adhesive composition, comprising:
(A) 10% to 30% by weight, based on the total weight of the adhesive composition, of an olefin block copolymer of ethylene and octene, having a melt index of 10 to 35 g/10 min @ 190°C, 2.16 kg, preferably 15 to 30 g/10 min @ 190°C, 2.16 kg, and
(B) a wax modified with carboxylic acid and/or carboxylic acid anhydride,
wherein the composition has a melt viscosity at 160°C from 2,000 to 3500 mPa.s, as determined using a Brookfield RVT-type viscometer (Spindle No. 27).

2. The composition of claim 1, wherein the wax (B) is a polyolefin wax modified with maleic acid and/or maleic acid anhydride.

3. The composition of claim 1 or claim 2, wherein the wax (B) is a polypropylene wax modified with maleic acid and/or maleic acid anhydride, or a polyethylene wax modified with maleic acid and/or maleic acid anhydride.

4. The composition of any one of claims 1 to 3, wherein the wax (B) has an acid number of no less than 10 mg KOH/g, preferably no less than 15 mg KOH/g.

5. The composition of any one of claims 1 to 4, wherein the composition further comprises (C) a tackifier, preferably selected from the group consisting of natural rosin, a modified rosin, a hydrogenated rosin, a glycerol ester of a natural rosin, a glycerol ester of a modified rosin, a pentaerythritol ester of a natural rosin, a pentaerythritol ester of a modified rosin, a pentaerythritol ester of a hydrogenated rosin, a copolymer of a natural terpene, hydrogenated derivatives of a copolymer of a hydrogenated terpene, a polyterpene resin, hydrogenated derivatives of a phenol-based modified terpene resin, an aliphatic petroleum hydrocarbon resin, hydrogenated derivatives of an aliphatic petroleum hydrocarbon resin, an aromatic petroleum hydrocarbon resin, hydrogenated derivatives of an aromatic petroleum hydrocarbon resin, a cyclic aliphatic petroleum hydrocarbon resin, and hydrogenated derivatives of a cyclic aliphatic petroleum hydrocarbon resin.

6. The composition of any one of claims 1 to 5, wherein the composition further comprises (D) an oil, preferably selected from the group consisting of paraffin oil, naphthene oil and aromatic oil.

7. The composition of any one of claims 1 to 6, wherein the composition further comprises (E) a stabilizer, preferably selected from the group consisting of antioxidants and ultraviolet absorbers.

8. The composition of any one of claims 1 to 7, wherein the composition further comprises one or more components selected from a filler, a pigment, a dye, and a rheology modifier.

9. The composition of any one of claims 1 to 8, wherein the olefin block copolymer of ethylene and octene (A) is comprised in an amount in the range of from 10% to 20% by weight, based on the total weight of the adhesive composition.

10. The composition of any one of claims 1 to 9, wherein the wax modified with carboxylic acid and/or carboxylic acid anhydride (B) is comprised in an amount in the range of from 0.1 to 10 % by weight, preferably 0.2 to 8 % by weight, each based on the total weight of the adhesive composition.

11. The composition of any one of claims 5 to 10, wherein the tackifier (C) is comprised in an amount in the range of from 30 to 80 % by weight, more preferably from 40 to 70 % by weight, each based on the total weight of the adhesive composition.

12. A laminate, comprising a first substrate, a second substrate, and an adhesive layer sandwiched therebetween, wherein the first and second substrates are independently of each other selected from a woven fabric, a nonwoven fabric, a rubber, a resin, papers and a polyolefin, and the adhesive layer is formed by the hot melt adhesive composition according to any one of claims 1 to 11.

13. A disposable article comprising the laminate of claim 12 or produced using the hot melt adhesive composition according to any one of claims 1 to 11.

14. The disposable article according to claim 13, including a disposable diaper, a sanitary napkin, a bed pad, a bandage, a surgical drape, a pet sheet, a hospital gown, and a surgical white garment.

## Patentansprüche

1. Schmelzklebstoffzusammensetzung, umfassend:
(A) zu 10 bis 30 Gew.-%, bezogen auf das Gesamtgewicht der Klebstoffzusammensetzung, ein Olefinblockcopolymer aus Ethylen und Octen, das einen Schmelzindex von 10 bis 35 g/10 min bei 190 °C, 2,16 kg, vorzugsweise 15 bis 30 g/10 min bei 190 °C, 2,16 kg, aufweist und
(B) ein Wachs, das mit Carbonsäure und/oder Carbonsäureanhydrid modifiziert ist,
wobei die Zusammensetzung eine Schmelzviskosität bei 160 °C von 2.000 bis 3.500 mPa.s aufweist, wie unter Verwendung eines Viskosimeters vom Typ Brookfield RVT (Spindel Nr. 27) bestimmt.

2. Zusammensetzung nach Anspruch 1, wobei das Wachs (B) ein Polyolefinwachs ist, das mit Maleinsäure und/oder Maleinsäureanhydrid modifiziert ist.

3. Zusammensetzung nach Anspruch 1 oder 2, wobei das Wachs (B) ein Polypropylenwachs, das mit Maleinsäure und/oder Maleinsäureanhydrid modifiziert ist, oder ein Polyethylenwachs ist, das mit Maleinsäure und/oder Maleinsäureanhydrid modifiziert ist.

4. Zusammensetzung nach einem der Ansprüche 1 bis 3, wobei das Wachs (B) eine Säurezahl von nicht weniger als 10 mg KOH/g, vorzugsweise nicht weniger als 15 mg KOH/g, aufweist.

5. Zusammensetzung nach einem der Ansprüche 1 bis 4, wobei die Zusammensetzung ferner (C) einen Klebrigmacher umfasst, der vorzugsweise aus der Gruppe ausgewählt ist, bestehend aus natürlichem Kolophonium, einem modifizierten Kolophonium, einem hydrierten Kolophonium, einem Glycerinester eines natürlichen Kolophoniums, einem Glycerinester eines modifizierten Kolophoniums, einem Pentaerythritester eines natürlichen Kolophoniums, einem Pentaerythritester eines modifizierten Kolophoniums, einem Pentaerythritester eines hydrierten Kolophoniums, einem Copolymer eines natürlichen Terpens, hydrierten Derivaten eines Copolymers eines hydrierten Terpens, einem Polyterpenharz, hydrierten Derivaten eines modifizierten Terpenharzes auf Phenolbasis, einem aliphatischen Petroleumkohlenwasserstoffharz, hydrierten Derivaten eines aliphatischen Petroleumkohlenwasserstoffharzes, einem aromatischen Petroleumkohlenwasserstoffharz, hydrierten Derivaten eines aromatischen Petroleumkohlenwasserstoffharzes, einem zyklischen aliphatischen Petroleumkohlenwasserstoffharz und hydrierten Derivaten eines zyklischen aliphatischen Petroleumkohlenwasserstoffharzes.

6. Zusammensetzung nach einem der Ansprüche 1 bis 5, wobei die Zusammensetzung ferner (D) ein Öl umfasst, das vorzugsweise aus der Gruppe ausgewählt ist, bestehend aus Paraffinöl, Naphtenöl und aromatischem Öl.

7. Zusammensetzung nach einem der Ansprüche 1 bis 6, wobei die Zusammensetzung ferner (E) einen Stabilisator umfasst, der vorzugsweise aus der Gruppe ausgewählt ist, bestehend aus Antioxidantien und Ultraviolettabsorbern.

8. Zusammensetzung nach einem der Ansprüche 1 bis 7, wobei die Zusammensetzung ferner eine oder mehrere Komponenten umfasst, die aus einem Füllstoff, einem Pigment, einem Farbstoff und einem Rheologiemodifikator ausgewählt sind.

9. Zusammensetzung nach einem der Ansprüche 1 bis 8, wobei das Olefinblockcopolymer aus Ethylen und Octen (A) in einer Menge im Bereich von 10 bis 20 Gew.-%, bezogen auf das Gesamtgewicht der Klebstoffzusammensetzung, enthalten ist.

10. Zusammensetzung nach einem der Ansprüche 1 bis 9, wobei das Wachs, das mit Carbonsäure und/oder Carbonsäureanhydrid (B) modifiziert ist, in einer Menge im Bereich von 0,1 bis 10 Gew.-%, vorzugsweise 0,2 bis 8 Gew.-%, jeweils bezogen auf das Gesamtgewicht der Klebstoffzusammensetzung, enthalten ist.

11. Zusammensetzung nach einem der Ansprüche 5 bis 10, wobei der Klebrigmacher (C) in einer Menge im Bereich von 30 bis 80 Gew.-%, stärker bevorzugt von 40 bis 70 Gew.-%, jeweils basierend auf dem Gesamtgewicht der Klebstoffzusammensetzung, enthalten ist.

12. Laminat, umfassend ein erstes Substrat, ein zweites Substrat und eine dazwischen eingeschobene Klebstoffschicht, wobei das erste und das zweite Substrat unabhängig voneinander aus einem Gewebe, einem Vlies, einem Gummi, einem Harz, Papieren und einem Polyolefin ausgewählt sind und die Klebstoffschicht durch die Schmelzklebstoffzusammensetzung nach einem der Ansprüche 1 bis 11 ausgebildet ist.

13. Einwegartikel, umfassend das Laminat nach Anspruch 12 oder der unter Verwendung der Schmelzklebstoffzusammensetzung nach einem der Ansprüche 1 bis 11 hergestellt ist.

14. Einwegartikel nach Anspruch 13, das eine Einwegwindel, eine Binde, eine Bettunterlage, einen Verband, ein Operationstuch, eine Haustierunterlage, ein Krankenhaushemd und ein weißes Operationskleidungsstück einschließt.

## Revendications

1. Composition d'adhésif thermofusible, comprenant :
(A) 10 % à 30 % en poids, sur la base du poids total de la composition d'adhésif, d'un copolymère à blocs oléfinique d'éthylène et d'octène, ayant un indice de fluidité de 10 à 35 g/10 min @ 190 °C, 2,16 kg, de préférence de 15 à 30 g/10 min @ 190 °C, 2,16 kg, et
(B) une cire modifiée par acide carboxylique et/ou anhydride d'acide carboxylique,
dans laquelle la composition a une viscosité à l'état fondu à 160 °C allant de 2 000 à 3 500 mPa.s, telle que déterminée à l'aide d'un viscosimètre Brookfield de type RVT (mobile n° 27).

2. Composition selon la revendication 1, dans laquelle la cire (B) est une cire de polyoléfine modifiée par acide maléique et/ou anhydride d'acide maléique.

3. Composition selon la revendication 1 ou la revendication 2, dans laquelle la cire (B) est une cire de polypropylène modifiée par acide maléique et/ou anhydride d'acide maléique, ou une cire de polyéthylène modifiée par acide maléique et/ou anhydride d'acide maléique.

4. Composition selon l'une quelconque des revendications 1 à 3, dans laquelle la cire (B) a un indice d'acide d'au moins 10 mg KOH/g, de préférence d'au moins 15 mg KOH/g.

5. Composition selon l'une quelconque des revendications 1 à 4, dans laquelle la composition comprend en outre (C) un agent collant, de préférence choisi dans le groupe constitué de colophane naturelle, une colophane modifiée, une colophane hydrogénée, un ester glycérique d'une colophane naturelle, un ester glycérique d'une colophane modifiée, un ester de pentaérythritol d'une colophane naturelle, un ester de pentaérythritol d'une colophane modifiée, un ester de pentaérythritol d'une colophane hydrogénée, un copolymère d'un terpène naturel, des dérivés hydrogénés d'un copolymère d'un terpène hydrogéné, une résine polyterpénique, des dérivés hydrogénés d'une résine terpénique modifiée à base de phénol, une résine d'hydrocarbure de pétrole aliphatique, des dérivés hydrogénés d'une résine d'hydrocarbure de pétrole aliphatique, une résine d'hydrocarbure de pétrole aromatique, des dérivés hydrogénés d'une résine d'hydrocarbure de pétrole aromatique, une résine d'hydrocarbure de pétrole aliphatique cyclique, et des dérivés hydrogénés d'une résine d'hydrocarbure de pétrole aliphatique cyclique.

6. Composition selon l'une quelconque des revendications 1 à 5, dans laquelle la composition comprend en outre (D) une huile, de préférence choisie dans le groupe constitué d'huile de paraffine, huile de naphtène et huile aromatique.

7. Composition selon l'une quelconque des revendications 1 à 6, dans laquelle la composition comprend en outre (E) un stabilisant, de préférence choisi dans le groupe constitué d'antioxydants et absorbeurs d'ultraviolets.

8. Composition selon l'une quelconque des revendications 1 à 7, dans laquelle la composition comprend en outre un ou plusieurs composants choisis parmi une charge, un pigment, un colorant, et un modificateur de rhéologie.

9. Composition selon l'une quelconque des revendications 1 à 8, dans laquelle le copolymère à blocs oléfinique d'éthylène et d'octène (A) est compris en une quantité dans la plage allant de 10 % à 20 % en poids, sur la base du poids total de la composition d'adhésif.

10. Composition selon l'une quelconque des revendications 1 à 9, dans laquelle la cire modifiée par acide carboxylique et/ou anhydride d'acide carboxylique (B) est comprise en une quantité dans la plage allant de 0,1 à 10 % en poids, de préférence de 0,2 à 8 % en poids, chacune sur la base du poids total de la composition d'adhésif.

11. Composition selon l'une quelconque des revendications 5 à 10, dans laquelle l'agent collant (C) est compris en une quantité dans la plage allant de 30 à 80 % en poids, plus préférablement de 40 à 70 % en poids, chacune sur la base du total de la composition d'adhésif.

12. Stratifié, comprenant un premier substrat, un second substrat, et une couche d'adhésif prise en sandwich entre eux, dans lequel les premier et second substrats sont, indépendamment l'un de l'autre, choisis parmi un tissu tissé, un tissu non tissé, un caoutchouc, une résine, des papiers et une polyoléfine, et la couche d'adhésif est formée par la composition d'adhésif thermofusible selon l'une quelconque des revendications 1 à 11.

13. Article jetable comprenant le stratifié selon la revendication 12 ou produit à l'aide de la composition d'adhésif thermofusible selon l'une quelconque des revendications 1 à 11.

14. Article jetable selon la revendication 13, y compris une couche jetable, une serviette hygiénique, une alèse, un bandage, un drap chirurgical, un drap pour animaux de compagnie, une blouse d'hôpital, et un vêtement blanc chirurgical.
